# EUROPÄISCHE PATENTSCHRIFT

(11) **EP 0 252 961 B1**
(45) Veröffentlichungstag und Bekanntmachung des Hinweises auf die Patenterteilung: **08.02.1995**
(21) Anmeldenummer: 87900721.9
(22) Anmeldetag: 18.12.1986
(51) Int. Cl.: C07D 241/12, C07D 241/18, C07D 241/24, C07D 213/55, C07D 213/30, C07D 213/16, C07D 213/26, C07D 213/57, C09K 19/34

(54) **HETEROCYCLISCHE VERBINDUNGEN ALS KOMPONENTEN FLÜSSIGKRISTALLINER PHASEN**
HETEROCYCLIC COMPOUNDS AS COMPONENTS OF LIQUID CRYSTAL PHASES
COMPOSES HETEROCYCLIQUES UTILISES COMME COMPOSANTS DE PHASES DE CRISTAUX LIQUIDES

(30) Priorität: 03.01.1986 DE 3600052
(43) Veröffentlichungstag der Anmeldung: 20.01.1988
(60) Teilanmeldung: 91119403.3
(73) Patentinhaber: MERCK PATENT GmbH, D-64271 Darmstadt (DE)
(72) Erfinder: GRAY, George, William, Cottingham HU16 4DY (GB); TOYNE, Kenneth, Johnson, Hull HU6 8QW (GB); LACEY, David, Hull HU8 9NW (GB); BURROW, Michael, Peter, Bristol BS8 4PW (GB); EIDENSCHINK, Rudolf, D-6115 Münster (DE); WÄCHTLER, Andreas, D-6103 Griesheim (DE); WEBER, Georg, D-6106 Erzhausen (DE)
(86) Internationale Anmeldenummer: EP8600760
(87) Internationale Veröffentlichungsnummer: WO8704158

(56) Entgegenhaltungen:
- EP-A- 0 164 721
- EP-A- 0 194 153
- WO-A-86/06401
- DE-A- 3 404 055
- GB-A- 2 161 808
- JP-A-60 149 564
- JP-A-60 163 865
- JP-A-60 199 882
- CHEMICAL ABSTRACTS, vol. 103, no. 25, 23rd December 1985, page 898, abstract-no. 215316p, Columbus, Ohio, US
- CHEMICAL ABSTRACTS, vol. 102, no. 9, 4th March 1985, page 604, abstract-no. 78913u, Columbus, Ohio, US

## Beschreibung

Die Erfindung betrifft heterocyclische Verbindungen der Formel I 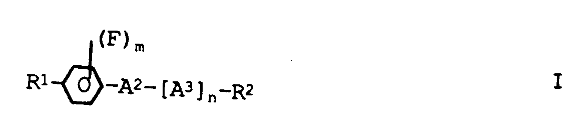
worin
- R¹ und R²: jeweils unabhängig voneinander eine Alkylgruppe mit 1-15 C-Atomen, worin auch eine oder zwei nicht benachbarte CH₂-Gruppen durch O-Atome und/oder -CO-Gruppen und/oder -O-CO-Gruppen und/oder -CO-O-Gruppen und/oder -O-COO-Gruppen und/oder -CHCN- und/oder -CH-Halogen-Gruppen ersetzt sein können, einer der Reste R¹ und R² auch F, Cl, Br oder CN,
- m: 1 oder mehr,
- A²: eine Pyrazin-2,5-diylgruppe (Pa) oder eine Pyridin-2,5-diylgruppe (Py),
- A³: eine 1,4-Cyclohexylengruppe oder eine 1 4-Phenylengruppe, die gegenbenenfalls auch durch F substituiert sein kann,
- n: 0 oder 1
bedeutet, mit der Maßgabe, daß für die Vertragsstaaten CH, DE, GB und Li Verbindungen der Formel 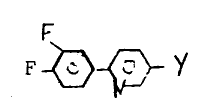
worin Y eine Alkylgruppe mit 1 bis 10 C-Atomen bedeutet, ausgeschlossen sind.

Der Einfachheit halber bedeuten im folgenden Cy eine 1,4-Cyclohexylengruppe, Phe eine 1,4-Phenylengruppe (die gegebenenfalls auch lateral durch F substituiert sein kann), Pa eine Pyrazin-2,5-diylgruppe und Py eine Pyridin-2,5-diylgruppe.

Ähnliche Verbindungen sind z.B. aus DE-C-25 35 046 bekannt. Die dort angegebenen Verbindungen enthalten im Gegensatz zu den vorliegenden keine Pyrazin-2,5-diyl- oder Pyridin-2,5-diylgruppe.

Ähnliche Pyridinverbindungen sind z. B. auch aus JP-A-60 149 564 und JP-A-60 163 865 bekannt. Ähnliche Pyrazinverbindungen sind beispielsweise in JP-A-60 199 882 beschrieben.

Weitere ähnliche Verbindungen sind aus JA-A-60163865; JA-A-60149564; JA-A-60199882; C.A. 103 (1985), s. 898, 215 316 p; C.A. 102 (1985), S. 604, 78913 u; EP-A-164721 und DE-A-3404055 bekannt sowie in den folgenden älteren, jedoch nicht vorpublizierten Schutzrechten beschrieben:
EP-A-194153; GB-A-2161808 und WO-A-86/06461.

Die Verbindungen der Formel I können wie ähnliche Verbindungen als Komponenten flüssigkristalliner Phasen verwendet werden, insbesondere für Displays, die auf dem Prinzip der verdrillten Zelle (TN-Displays), dem Guest-Host-Effekt, dem Effekt der Deformation aufgerichteter Phasen, dem Effekt der dynamischen Streuung oder dem SSFLC-Prinzip beruhen.

Der Erfindung lag die Aufgabe zugrunde, neue stabile flüssigkristalle oder mesogene Verbindungen aufzufinden, die als Komponenten flüssigkristalliner Phasen geeignet sind.

Es wurde gefunden, daß die Verbindungen der Formel I als Komponenten flüssigkristalliner Phasen vorzüglich geeignet sind. Insbesondere sind mit ihrer Hilfe stabile flüssigkristalline Phasen für TN-Displays mit hohen Multiplexraten, breiten Arbeitsbereichen und vorteilhaften elastischen Eigenschaften herstellbar. Weiterhin zeigen diese Phasen ausgeprägtes nematogenes Verhalten und günstige Schaltzeiten, insbesondere bei tiefen Temperaturen. Ferner eignen sich Verbindungen der Formel I, insbesondere optisch aktive Verbindungen der Formel I, als Komponenten ferroelektrischer Flüssigkristallphasen beispielsweise für Displays nach dem SSFLC-Prinzip.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie oder andere Parameter eines solchen Dielektrikums zu optimieren. Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I sowie Flüssigkristallanzeigeelemente, insbesondere elektrooptische Anzeigeelemente, die derartige Phasen enthalten.

Gegenstand der Erfindung ist auch ein ferroelektrisches Display nach Clark und Lagerwall, enthaltend Verbindungen der Formel I. Bevorzugt sind dabei Verbindungen der Formel I, in denen A³ 1-4 Phenylen bedeutet. R¹ und R² stellen dabei bevorzugt Alkyl- oder Alkoxygruppen dar, vorzugsweise mit jeweils 5 - 15 C-Atomen. R¹ und R² besitzen zusammen vorzugsweise mindestens 10 C-Atome.

Vor- und nachstehend haben R¹, R², A², A³, m und n die angegebene Bedeutung, sofern nicht ausdrücklich etwas anderes vermerkt ist. Nachstehend bedeutet A¹ ein Ringsystem der Formel

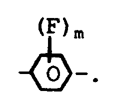

Die Verbindungen der Formel I umfassen dementsprechend Verbindungen der Teilformeln Ia (mit zwei Ringen) und Ic (mit drei Ringen):

R¹-A¹-A²-R² Ia

R¹-A¹-A²-A³-R² Ic

Darunter sind diejenigen der Teilformel Ic besonders bevorzugt.

Die bevorzugten Verbindungen der Teilformel Ia umfassen solche der Teilformeln Iaa und Iab ; wobei PheX lateral fluoriertes 1,4-Phenylen ist: 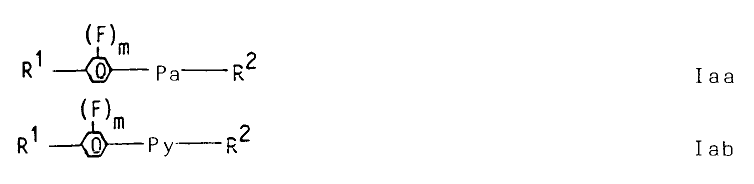
Darunter sind diejenigen der Teilformel Iab besonders bevorzugt.

Die bevorzugten Verbindungen der Teilformel Ic umfassen solche der Teilformeln Ica und Ich wobei Phe unsubstituiertes und PheX lateral fluoriertes 1,4-Phenylen ist: 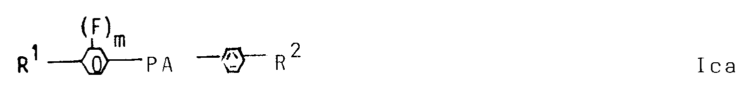 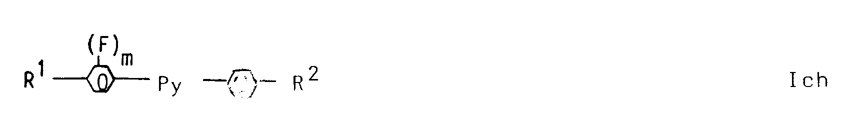
Darunter sind diejenigen der Teilformel Ich besonders bevorzugt.

In den Verbindungen der vor- und nachstehenden Formeln bedeuten R¹ und R² vorzugsweise n-Alkyl, -O-n-Alkyl, -OCO-n-Alkyl, -COO-n-Alkyl oder -OCOO-n-Alkyl oder Oxaalkyl. In der n-Alkylgruppe kann auch eine-CH₂-Gruppe durch -CH-CH₃-, -CH-Halogen oder -CH-CN ersetzt sein. Besonders bevorzugt sind Verbindungen der Formel I, worin eine der Gruppen R¹ und R² Alkyl und die andere Alkyl, Alkoxy oder Fluor, insbesondere bevorzugt Alkyl, bedeutet.

A³ ist Cy oder Phe. Insbesondere bevorzugt hat A³ die Bedeutung von lateral fluoriertem 1,4-Phenylen.

Besonders bevorzugt sind Verbindungen der Formel I, worin A² Py bedeutet und die andere Gruppe A³ 1,4-Phenylen bedeutet.

Besonders bevorzugt ist Monofluorsubstitution an einer 1,4-Phenylengruppe.
- n: ist vorzugsweise 1.

Die Alkylreste in den Gruppen R¹ und/oder R² können geradkettig oder verzweigt sein. Vorzugsweise sind sie geradkettig, haben 2, 3, 4, 5, 6, 7, 8, 9, 10, 11 oder 12 C-Atome und bedeuten demnach bevorzugt Ethyl, Propyl, Butyl, Pentyl, Hexyl, Heptyl, Octyl, Nonyl, Decyl, Undecyl oder Dodecyl, ferner Methyl, Tridecyl, Tetradecyl oder Pentadecyl.

Falls R¹ und/oder R² Alkylreste bedeuten, in denen eine ("Alkoxy" bzw. "Oxaalkyl") oder zwei ("Alkoxyalkoxy" bzw. "Dioxaalkyl") CH₂-Gruppen durch O-Atome ersetzt sind, so können sie geradkettig oder verzweigt sein. Vorzugsweise sind sie geradkettig, haben 2, 3, 4, 5, 6 oder 7 C-Atome und bedeuten demnach bevorzugt Ethoxy, Propoxy, Butoxy, Pentoxy, Hexoxy, Heptoxy, 2-Oxapropyl (= Methoxymethyl), 2-Ethoxymethyl oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methoxy, Octoxy, Nonoxy, Decoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3- 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Verbindungen der Formel I sowie der vor- und nachstehenden Teilformeln mit verzweigten Flügelgruppen R¹ bzw. R² können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste R¹ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl, 2-Octyloxy, 2-Chlorpropionyloxy, 2-Chlor-3-methylbutyryloxy, 2-Chlor-4-methylvaleryloxy, 2-Chlor-3-methylvaleryloxy, 2-Methyl-3-oxapentyl, 2-Methyl-3-oxa-hexyl. Optisch aktive Verbindungen der Formel I eignen sich als Komponenten chiral smektischer Flüssigkristallphasen und sind ferner auch in nematischen Materialien z.B. zur Vermeidung von reverse twist geeignet.

Besonders bevorzugte Pyrazinverbindungen sind solche, die als A³ ein lateral fluoriertes 1,4-Phenylen enthalten.

Bei den Pyridinverbindungen der Formel I sind diejenigen bevorzugt, in denen n 1 ist. Weiterhin enthalten bevorzugte Pyridinverbindungen mindestens eine 1,4-Cyclohexylengruppe oder eine lateral fluorierte 1,4-Phenylengruppe.

Unter den Verbindungen der Formel I sowie der vor- und nachstehenden Teilformeln sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln I1 bis I8 worin PheX 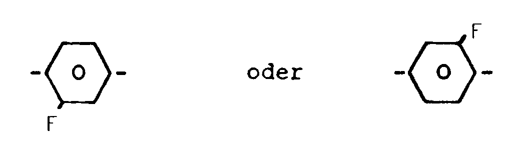
bedeutet.

Alkyl-Phe-Pa-PheX-Alkyl I1

Alkyl-Pa-PheX-Alkyl I2

Alkyl-Py-PheX-Alkyl I3

Alkyl-Py-PheX-Alkoxy I4

Alkoxy-Py-PheX-Alkyl I5

Alkyl-Py-PheX-CN I6

Alkoxy-Py-PheX-Alkyl I7

Alkyl-PheX-Py-Cy-Alkyl I7

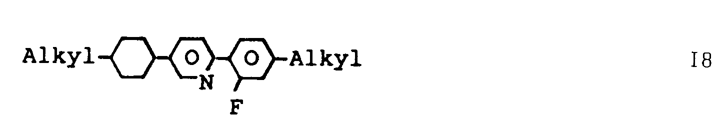

In den vorstehenden Formeln I1 bis I8 bedeutet Alkyl vorzugsweise eine Alkylgruppe mit 2 bis 10 C-Atomen und Alkoxy eine Alkoxygruppe mit 2 bis 12 C-Atomen. Weiterhin bevorzugt sind Verbindungen der Formeln I1 bis I8, in denen an Stelle von Alkoxy eine 2-Oxaalkylgruppe mit 2 bis 12 C-Atomen vorhanden ist.

Unter den Verbindungen der Formel I sind diejenigen Stereoisomeren bevorzugt, in denen die gesättigten Ringe (Cy) trans-1,4-disubstituiert sind.

Die Verbindungen der Formel I werden nach an sich bekannten Methoden hergestellt, wie sie in der Literatur (z.B. in den Standardwerken wie Houben Weyl), Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht erwähnten Varianten Gebrauch machen.

Der Fachmann kann durch Routinemethoden entsprechende Synthesemethoden aus dem Stand der Technik entnehmen (z.B. DE-A-23 44 732, DE-A-24 50 088, DE-A-24 29 093, DE-A-25 02 904, DE-A-26 36 684, DE-A-27 01 591 und DE-A-27 52 975 betreffend Verbindungen mit 1,4-Cyclohexylen und 1,4-Phenylen-Gruppen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanrings einen Cyclohexenring oder Cyclohexanonring und/oder an Stelle einer -CH₂CH₂-Gruppe eine -CH=CH-Gruppe und/oder an Stelle einer -CH₂-Gruppe eine -CO-Gruppe und/oder an Stelle eines H-Atomes eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen 0° und 200° sowie Drucken zwischen 1 und 200 bar in einem inerten Lösungsmittel, z.B. einem Alkohol wie Methanol, Ethanol oder Isopropranol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. PtO₂, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen 80 und 120 °C) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen 100 und 200 °C) zu den entsprechenden Verbindungen der Formel I, die Alkylgruppen enthalten, reduziert werden.

Weiterhin sind Reduktionen mit komplexen Hydriden möglich. Beispielsweise können Arylsulfonyloxygruppen mit LiAlH₄ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100 °C. Doppelbindungen können (auch in Gegenwart von CN-Gruppen!) mit NaBH₄ oder Tributylzinnhydrid in Methanol hydriert werden.

Ester der Formel I (R¹ und/oder R² = Alkyl, worin eine oder zwei CH₂-Gruppen durch -O-CO- und/oder -CO-O-Gruppen ersetzt sind) können auch durch Veresterung entsprechender Carbonsäure (oder ihren reaktionsfähigen Derivaten) mit Alkoholen bzw. Phenolen (oder ihrer reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen -50° und +250°, vorzugsweise zwischen -20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa -25° und +20°.

Zur Herstellung von Nitrilen der Formel I (worin R¹ oder R² CN bedeutet) können entsprechende Säureamide dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie SOCl₂, PCl₃, PCl₅, POCl₃, SO₂Cl₂, COCl₂, ferner P₂O₅, P₂S₅, AlCl₃ (z.B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen 0° und 150° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie Tetramethylsulfon bei Temperaturen zwischen 80° und 150°, vorzugsweise bei 120°. Nach üblicher Aufarbeitung kann man direkt die Nitrile isolieren.

Ether der Formel I (worin R¹ und/oder R² eine Alkylgruppe bedeutet, worin eine oder zwei CH₂-Gruppen durch O-Atome ersetzt sind) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂CO₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfonat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen 20° und 100°.

Zur Herstellung von Nitrilen der Formel I (worin R¹ oder R² CN bedeutet) können auch entsprechende Chlor- oder Bromverbindungen der Formel I mit einem Cyanid umgesetzt werden, zweckmäßig mit einem Metallcyanid wie NaCN, KCN oder Cu₂(CN)₂, z.B. in Gegenwart von Pyridin in einem inerten Lösungsmittel wie DMF oder N-Methylpyrrolidon bei Temperaturen zwischen 20° und 200°.

Zur Herstellung von lateral substituierten Fluor-Verbindungen der Formel I (worin A¹ und/oder A³ Aromaten bedeuten, R¹ und R² die üblichen Bedeutungen haben können) können entsprechende Aminoverbindungen (erhältlich durch Reduktion der entsprechenden Nitroverbindungen nach Standardmethoden, z.B. durch katalytische Hydrierung, durch Behandlung mit wäßrigem Dithionit oder mit Zinn-(II)-chlorid und Salzsäure) mit Natriumnitrit und mit Tetrafluorborsäure (zur Einfuhrung eines F-Atoms) zu den Diazoniumsalzen umgesetzt werden, die dann bei Temperaturen von 100°-250° thermisch zersetzt werden.

Eine Base der Formel I kann mit einer Säure in das zugehörige Säureadditionssalz überführt werden. Für diese Umsetzung können anorganische Säuren verwendet werden, z.B. Schwefelsäure, Salpetersäure, Halogenwasserstoffsäure wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und-disulfonsäuren, Laurylschwefelsäure.

Umgekehrt ist es möglich, aus einem Säureadditionssalz einer Verbindung der Formel I die Base der Formel I durch Behandeln mit einer Base freizusetzen, z.B. mit einer starken anorganischen Base wie KOH oder NaOH.

Die erfindungsgemäßen flüssigkristallinen Phasen bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexancarbonsäurephenyl- oder cyclohexylester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, Phenyl- oder Cyclohexyl-1,3-dithiane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel II charakterisieren,

R'-L-G-E-R'' II

worin L und E je ein carbo- oder heterocyclisches Ringsystem aus der aus 1,4-disubstituierten Benzol und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | -CH=CH- | -N(O)=N- |
| | -CH=CY- | -CH=N(O)- |
| | -C=C- | -CH₂-CH₂- |
| | -CO-O- | -CH₂-O- |
| | -CO-S- | -CH₂-S- |
| | -CH=N- | -COO-Phe-COO- |

oder eine C-C-Einfachbindung, Y Halogen, vorzugsweise Chlor, oder CN, und R' und R'' Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, NO₂, CH₃, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden herstellbar.

Die erfindungsgemäßen Phasen enthalten etwa 0,1 bis 99, vorzugsweise 10 bis 95 %, einer oder mehrerer Verbindungen der Formel I.

Weiterhin bevorzugt sind erfindungsgemäße Dielektrika enthaltend 0,1 bis 40, vorzugsweise 0,5 bis 30 %, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol.Cryst.Liq.Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-A-22 09 127, DE-A-22 40 854, DE-A-23 21 632, DE-A-23 38 281, DE-A-24 50 088, DE-A-26 37 430, DE-A-28 53 728 und DE-A-29 02 177 beschrieben.

Die folgenden Beispiele sollen die Erfindung erläutern. C, S, Ch, N und I bedeuten kristalliner, smektischer, cholesterischer, nematischer und isotroper Zustand. Die Zahlen dazwischen stellen die Übergangstemperaturen dar. "F". bedeutet Schmelzpunkt. Vor- und nachstehend bedeuten Prozentangaben Gewichtsprozent; alle Temperaturangaben sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

### Vergleichsbeispiel 1:

Zu einer Lösung von 0,019 mol 3-Chlor-1-[4-(trans-4-pentylcyclohexylethyl)phenyl]propan-1-on (F. 73-74°, herstellbar durch a) Umsetzung von Benzol mit trans-4-Pentylcyclohexylacetylchlorid nach Friedel-Craft, b) anschließende Reduktion nach Huang-Minlon zu 1-(trans-4-Pentylcyclohexyl)-2-phenylethan (Siedepunkt: 125°/0,3 mmHg) und c) Umsetzung dieser Verbindung mit 3-Chlorpropanoylchlorid in CH₂Cl₂ und AlCl₃) in 5 ml Tetrahydrofuran gibt man unter Rühren 0,02 mol Triethylamin. Man rührt eine Stunde und gibt dann 0,019 mol 1-Piperidinopenten (Siedepunkt: 101-104°/18 mmHg, herstellbar nach G. Stork et al., J. Amer. Chem. Soc. 1963, 85, 207 und C. Mannich und H. Davidsen, Chem. Ber. 1936, 69, 2106) in 5 ml THF zu und rührt 72 Stunden.

Das Lösungsmittel wird entfernt und der Rückstand in Ethanol-Wasser (1:1, 10 ml) aufgenommen und mit 0,059 mol Hydroxylaminhydrochlorid unter Rühren 72 Stunden lang erhitzt.

Das Lösungsmittel wird abgedampft und der Rückstand mit NaOH-Lösung (10 %, 100 ml) und Ether (50 ml) behandelt. Die organische Phase wird wie üblich aufgearbeitet und man erhält nach Reinigung durch Säulenchromatographie (neutrales Aluminiumoxid, Elutionsmittel Petrolether-Chloroform) und Umkristallisation 2-p-[2'-(trans-4-Pentylcyclohexyl)ethyl]phenyl-5-propylpyridin mit C 36° S_{G} 53° S_{B} 100° N 150° I (vgl. auch C. Botteghi et al., Synth. Comm. 1979, 9(2), 69).

### Vergleichsbeispiel 2

Ein Gemisch aus 5,0 mmol 1-(trans-4-Hexylcyclohexyl)prop-2-en-1-on [darstellbar durch a) Umsetzung von trans-4-Hexylcyclohexancarbonsäurechlorid mit AlCl₃ in Dichlormethan und Einleiten von Ethen zu 1-(trans-4-Hexylcyclohexyl)-3-chlorpropan-1-on und b) Umsetzung und Erhitzen mit Triethylamin in THF, Siedepunkt 60-63°/0,03 mmHg] und 5,02 mmol 1-Piperidino-2-(4-propylphenyl)ethen (darstellbar durch Umsetzung von 4-Propylphenylessigsäure mit einem Gemisch aus Di-isopropylamin und n-Butyllithium in THF und 4-(Methoxymethylen)-piperidiniummethylsulfat analog R. Knorr et al., Synthesis 1983, 785) wird in 10 ml THF gelöst und bei Raumtemperatur 72 Stunden gerührt (nach C. Botteghi et al., Synth. Comm., 1979, 9(2), 69). Das Lösungsmittel wird entfernt und der Rückstand in Wasser-Ethanol (1:1, 3 ml) aufgenommen, 22,3 mmol Hydroxylaminhydrochlorid zugegeben und 24 Stunden erhitzt. Das Lösungsmittel wird entfernt und der Rückstand mit Ether aufgenommen.

Die organische Phase wird wie üblich aufgearbeitet und man erhält nach Reinigung durch Säulenchromatographie an Al₂O₃ und Umkristallisation 2-(trans-4-Hexylcyclohexyl)-5-(4-propylphenyl)pyridin mit C 94° S_{B} 127° S_{A} 134° N 147° I.

### Beispiel 1

Analog Vergleichsbeispiel 1 erhält man aus 1-(2-Fluor-4-propylphenyl)-3-chlorpropan-1-on (darstellbar aus 2-Fluorpropylbenzol und 3-Chlorpropanoylchlorid nach Friedel-Crafts) und 1-Piperidino-2-(trans-4-pentylcyclohexyl)-ethen (nach Synthesis, 1983, 785 herstellbar) das entsprechende 5-(trans-4-Pentylcyclohexyl)-2-(2-fluor-4-propylphenyl)pyridin.

Analog werden hergestellt:
5-(trans-4-Pentylcyclohexyl)-2-(2-fluor-4-ethylphenyl)pyridin
5-(trans-4-Pentylcyclohexyl)-2-(2-fluor-4-butylphenyl)pyridin
5-(trans-4-Pentylcyclohexyl)-2-(2-fluor-4-pentylphenyl)pyridin
5-(trans-4-Pentylcyclohexyl)-2-(2-fluor-4-hexylphenyl)pyridin
5-(trans-4-Pentylcyclohexyl)-2-(2-fluor-4-heptylphenyl)pyridin
5-(trans-4-Pentylcyclohexyl)-2-(2-fluor-4-octylphenyl)pyridin
5-(trans-4-Pentylcyclohexyl)-2-(2-fluor-4-nonylphenyl)pyridin
5-(trans-4-Propylcyclohexyl)-2-(2-fluor-4-propylphenyl)pyridin
5-(trans-4-Propylcyclohexyl)-2-(2-fluor-4-ethylphenyl)pyridin
5-(trans-4-Propylcyclohexyl)-2-(2-fluor-4-butylphenyl)pyridin
5-(trans-4-Propylcyclohexyl)-2-(2-fluor-4-pentylphenyl)pyridin, C 47° N 149° I
5-(trans-4-Propylcyclohexyl)-2-(2-fluor-4-hexylphenyl)pyridin
5-(trans-4-Propylcyclohexyl)-2-(2-fluor-4-heptylphenyl)pyridin
5-(trans-4-Propylcyclohexyl)-2-(2-fluor-4-octylphenyl)pyridin
5-(trans-4-Propylcyclohexyl)-2-(2-fluor-4-nonylphenyl)pyridin
5-(trans-4-Butylcyclohexyl)-2-(2-fluor-4-propylphenyl)pyridin
5-(trans-4-Butylcyclohexyl)-2-(2-fluor-4-ethylphenyl)pyridin
5-(trans-4-Butylcyclohexyl)-2-(2-fluor-4-butylphenyl)pyridin
5-(trans-4-Butylcyclohexyl)-2-(2-fluor-4-pentylphenyl)pyridin
5-(trans-4-Butylcyclohexyl)-2-(2-fluor-4-hexylphenyl)pyridin
5-(trans-4-Butylcyclohexyl)-2-(2-fluor-4-heptylphenyl)pyridin
5-(trans-4-Butylcyclohexyl)-2-(2-fluor-4-octylphenyl)pyridin
5-(trans-4-Butylcyclohexyl)-2-(2-fluor-4-nonylphenyl)pyridin
5-(trans-4-Hexylcyclohexyl)-2-(2-fluor-4-propylphenyl)pyridin
5-(trans-4-Hexylcyclohexyl)-2-(2-fluor-4-ethylphenyl)pyridin
5-(trans-4-Hexylcyclohexyl)-2-(2-fluor-4-butylphenyl)pyridin
5-(trans-4-Hexylcyclohexyl)-2-(2-fluor-4-pentylphenyl)pyridin
5-(trans-4-Hexylcyclohexyl)-2-(2-fluor-4-hexylphenyl)pyridin
5-(trans-4-Hexylcyclohexyl)-2-(2-fluor-4-heptylphenyl)pyridin
5-(trans-4-Hexylcyclohexyl)-2-(2-fluor-4-octylphenyl)pyridin
5-(trans-4-Hexylcyclohexyl)-2-(2-fluor-4-nonylphenyl)pyridin
5-(trans-4-Heptylcyclohexyl)-2-(2-fluor-4-propylphenyl)pyridin
5-(trans-4-Heptylcyclohexyl)-2-(2-fluor-4-ethylphenyl)pyridin
5-(trans-4-Heptylcyclohexyl)-2-(2-fluor-4-butylphenyl)pyridin
5-(trans-4-Heptylcyclohexyl)-2-(2-fluor-4-pentylphenyl)pyridin
5-(trans-4-Heptylcyclohexyl)-2-(2-fluor-4-hexylphenyl)pyridin
5-(trans-4-Heptylcyclohexyl)-2-(2-fluor-4-heptylphenyl)pyridin
5-(trans-4-Heptylcyclohexyl)-2-(2-fluor-4-octylphenyl)pyridin
5-(trans-4-Heptylcyclohexyl)-2-(2-fluor-4-nonylphenyl)pyridin
5-(trans-4-Octylcyclohexyl)-2-(2-fluor-4-propylphenyl)pyridin
5-(trans-4-Octylcyclohexyl)-2-(2-fluor-4-ethylphenyl)pyridin
5-(trans-4-Octylcyclohexyl)-2-(2-fluor-4-butylphenyl)pyridin
5-(trans-4-Octylcyclohexyl)-2-(2-fluor-4-pentylphenyl)pyridin
5-(trans-4-Octylcyclohexyl)-2-(2-fluor-4-hexylphenyl)pyridin
5-(trans-4-Octylcyclohexyl)-2-(2-fluor-4-heptylphenyl)pyridin
5-(trans-4-Octylcyclohexyl)-2-(2-fluor-4-octylphenyl)pyridin
5-(trans-4-Octylcyclohexyl)-2-(2-fluor-4-nonylphenyl)pyridin
5-(trans-4-Ethylcyclohexyl)-2-(2-fluor-4-propylphenyl)pyridin
5-(trans-4-Ethylcyclohexyl)-2-(2-fluor-4-ethylphenyl)pyridin
5-(trans-4-Ethylcyclohexyl)-2-(2-fluor-4-butylphenyl)pyridin
5-(trans-4-Ethylcyclohexyl)-2-(2-fluor-4-pentylphenyl)pyridin
5-(trans-4-Ethylcyclohexyl)-2-(2-fluor-4-hexylphenyl)pyridin
5-(trans-4-Ethylcyclohexyl)-2-(2-fluor-4-heptylphenyl)pyridin
5-(trans-4-Ethylcyclohexyl)-2-(2-fluor-4-octylphenyl)pyridin
5-(trans-4-Ethylcyclohexyl)-2-(2-fluor-4-nonylphenyl)pyridin

### Beispiel 2:

Analog Beispiel 1 erhält man aus 1-(2-Fluor-4-pentylphenyl)-3-chlorpropan-1-on und 1-Piperidino-2-(4-propylphenyl)-ethen (analoge Darstellung wie in Vergleichsbeispiel 2) 2-(2-Fluor-4-pentylphenyl)-5-(4-propylphenyl)-pyridin.

Analog werden hergestellt:
2-(2-Fluor-4-pentylphenyl)-5-(4-ethylphenyl)pyridin
2-(2-Fluor-4-pentylphenyl)-5-(4-butylphenyl)pyridin
2-(2-Fluor-4-pentylphenyl)-5-(4-pentylphenyl)pyridin
2-(2-Fluor-4-pentylphenyl)-5-(4-hexylphenyl)pyridin
2-(2-Fluor-4-pentylphenyl)-5-(4-heptylphenyl)pyridin
2-(2-Fluor-4-pentylphenyl)-5-(4-octylphenyl)pyridin
2-(2-Fluor-4-pentylphenyl)-5-(4-nonylphenyl)pyridin
2-(2-Fluor-4-pentylphenyl)-5-(4-ethoxyphenyl)pyridin
2-(2-Fluor-4-pentylphenyl)-5-(4-propoxyphenyl)pyridin
2-(2-Fluor-4-pentylphenyl)-5-(4-butoxyphenyl)pyridin
2-(2-Fluor-4-pentylphenyl)-5-(4-pentoxyphenyl)pyridin
2-(2-Fluor-4-pentylphenyl)-5-(4-hexoxyphenyl)pyridin
2-(2-Fluor-4-pentylphenyl)-5-(4-heptoxyphenyl)pyridin
2-(2-Fluor-4-pentylphenyl)-5-(4-octoxyphenyl)pyridin
2-(2-Fluor-4-pentylphenyl)-5-(4-nonoxyphenyl)pyridin
2-(2-Fluor-4-propylphenyl)-5-(4-propylphenyl)pyridin
2-(2-Fluor-4-propylphenyl)-5-(4-ethylphenyl)pyridin
2-(2-Fluor-4-propylphenyl)-5-(4-butylphenyl)pyridin
2-(2-Fluor-4-propylphenyl)-5-(4-hexylphenyl)pyridin
2-(2-Fluor-4-propylphenyl)-5-(4-heptylphenyl)pyridin
2-(2-Fluor-4-propylphenyl)-5-(4-octylphenyl)pyridin
2-(2-Fluor-4-propylphenyl)-5-(4-nonylphenyl)pyridin
2-(2-Fluor-4-propylphenyl)-5-(4-ethoxyphenyl)pyridin
2-(2-Fluor-4-propylphenyl)-5-(4-propoxyphenyl)pyridin
2-(2-Fluor-4-propylphenyl)-5-(4-butoxyphenyl)pyridin
2-(2-Fluor-4-propylphenyl)-5-(4-pentoxyphenyl)pyridin
2-(2-Fluor-4-propylphenyl)-5-(4-hexoxyphenyl)pyridin
2-(2-Fluor-4-propylphenyl)-5-(4-heptoxyphenyl)pyridin
2-(2-Fluor-4-propylphenyl)-5-(4-octoxyphenyl)pyridin
2-(2-Fluor-4-propylphenyl)-5-(4-nonoxyphenyl)pyridin
2-(2-Fluor-4-ethylphenyl)-5-(4-propylphenyl)pyridin
2-(2-Fluor-4-ethylphenyl)-5-(4-ethylphenyl)pyridin
2-(2-Fluor-4-ethylphenyl)-5-(4-butylphenyl)pyridin
2-(2-Fluor-4-ethylphenyl)-5-(4-pentylphenyl)pyridin
2-(2-Fluor-4-ethylphenyl)-5-(4-hexylphenyl)pyridin
2-(2-Fluor-4-ethylphenyl)-5-(4-heptylphenyl)pyridin
2-(2-Fluor-4-ethylphenyl)-5-(4-octylphenyl)pyridin
2-(2-Fluor-4-ethylphenyl)-5-(4-nonylphenyl)pyridin
2-(2-Fluor-4-ethylphenyl)-5-(4-ethoxyphenyl)pyridin
2-(2-Fluor-4-ethylphenyl)-5-(4-propoxyphenyl)pyridin
2-(2-Fluor-4-ethylphenyl)-5-(4-butoxyphenyl)pyridin
2-(2-Fluor-4-ethylphenyl)-5-(4-pentoxyphenyl)pyridin
2-(2-Fluor-4-ethylphenyl)-5-(4-hexoxyphenyl)pyridin
2-(2-Fluor-4-ethylphenyl)-5-(4-heptoxyphenyl)pyridin
2-(2-Fluor-4-ethylphenyl)-5-(4-octoxyphenyl)pyridin
2-(2-Fluor-4-ethylphenyl)-5-(4-nonoxyphenyl)pyridin
2-(2-Fluor-4-butylphenyl)-5-(4-propylphenyl)pyridin
2-(2-Fluor-4-butylphenyl)-5-(4-ethylphenyl)pyridin
2-(2-Fluor-4-butylphenyl)-5-(4-butylphenyl)pyridin
2-(2-Fluor-4-butylphenyl)-5-(4-pentylphenyl)pyridin
2-(2-Fluor-4-butylphenyl)-5-(4-hexylphenyl)pyridin
2-(2-Fluor-4-butylphenyl)-5-(4-heptylphenyl)pyridin
2-(2-Fluor-4-butylphenyl)-5-(4-octylphenyl)pyridin
2-(2-Fluor-4-butylphenyl)-5-(4-nonylphenyl)pyridin
2-(2-Fluor-4-butylphenyl)-5-(4-ethoxyphenyl)pyridin
2-(2-Fluor-4-butylphenyl)-5-(4-propoxyphenyl)pyridin
2-(2-Fluor-4-butylphenyl)-5-(4-butoxyphenyl)pyridin
2-(2-Fluor-4-butylphenyl)-5-(4-pentoxyphenyl)pyridin
2-(2-Fluor-4-butylphenyl)-5-(4-hexoxyphenyl)pyridin
2-(2-Fluor-4-butylphenyl)-5-(4-heptoxyphenyl)pyridin
2-(2-Fluor-4-butylphenyl)-5-(4-octoxyphenyl)pyridin
2-(2-Fluor-4-butylphenyl)-5-(4-nonoxyphenyl)pyridin
2-(2-Fluor-4-hexylphenyl)-5-(4-propylphenyl)pyridin
2-(2-Fluor-4-hexylphenyl)-5-(4-ethylphenyl)pyridin
2-(2-Fluor-4-hexylphenyl)-5-(4-butylphenyl)pyridin
2-(2-Fluor-4-hexylphenyl)-5-(4-hexylphenyl)pyridin
2-(2-Fluor-4-hexylphenyl)-5-(4-pentylphenyl)pyridin
2-(2-Fluor-4-hexylphenyl)-5-(4-heptylphenyl)pyridin
2-(2-Fluor-4-hexylphenyl)-5-(4-octylphenyl)pyridin
2-(2-Fluor-4-hexylphenyl)-5-(4-nonylphenyl)pyridin
2-(2-Fluor-4-hexylphenyl)-5-(4-ethoxyphenyl)pyridin
2-(2-Fluor-4-hexylphenyl)-5-(4-propoxyphenyl)pyridin
2-(2-Fluor-4-hexylphenyl)-5-(4-butoxyphenyl)pyridin
2-(2-Fluor-4-hexylphenyl)-5-(4-pentoxyphenyl)pyridin
2-(2-Fluor-4-hexylphenyl)-5-(4-hexoxyphenyl)pyridin
2-(2-Fluor-4-hexylphenyl)-5-(4-heptoxyphenyl)pyridin
2-(2-Fluor-4-hexylphenyl)-5-(4-octoxyphenyl)pyridin
2-(2-Fluor-4-hexylphenyl)-5-(4-nonoxyphenyl)pyridin
2-(2-Fluor-4-heptylphenyl)-5-(4-propylphenyl)pyridin
2-(2-Fluor-4-heptylphenyl)-5-(4-ethylphenyl)pyridin
2-(2-Fluor-4-heptylphenyl)-5-(4-butylphenyl)pyridin
2-(2-Fluor-4-heptylphenyl)-5-(4-pentylphenyl)pyridin
2-(2-Fluor-4-heptylphenyl)-5-(4-hexylphenyl)pyridin
2-(2-Fluor-4-heptylphenyl)-5-(4-heptylphenyl)pyridin
2-(2-Fluor-4-heptylphenyl)-5-(4-octylphenyl)pyridin
2-(2-Fluor-4-heptylphenyl)-5-(4-nonylphenyl)pyridin
2-(2-Fluor-4-heptylphenyl)-5-(4-ethoxyphenyl)pyridin
2-(2-Fluor-4-heptylphenyl)-5-(4-propoxyphenyl)pyridin
2-(2-Fluor-4-heptylphenyl)-5-(4-butoxyphenyl)pyridin
2-(2-Fluor-4-heptylphenyl)-5-(4-pentoxyphenyl)pyridin
2-(2-Fluor-4-heptylphenyl)-5-(4-hexoxyphenyl)pyridin
2-(2-Fluor-4-heptylphenyl)-5-(4-heptoxyphenyl)pyridin
2-(2-Fluor-4-heptylphenyl)-5-(4-octoxyphenyl)pyridin
2-(2-Fluor-4-heptylphenyl)-5-(4-nonoxyphenyl)pyridin
2-(2-Fluor-4-octylphenyl)-5-(4-propylphenyl)pyridin
2-(2-Fluor-4-octylphenyl)-5-(4-ethylphenyl)pyridin
2-(2-Fluor-4-octylphenyl)-5-(4-butylphenyl)pyridin
2-(2-Fluor-4-octylphenyl)-5-(4-pentylphenyl)pyridin
2-(2-Fluor-4-octylphenyl)-5-(4-hexylphenyl)pyridin
2-(2-Fluor-4-octylphenyl)-5-(4-heptylphenyl)pyridin
2-(2-Fluor-4-octylphenyl)-5-(4-octylphenyl)pyridin
2-(2-Fluor-4-octylphenyl)-5-(4-nonylphenyl)pyridin
2-(2-Fluor-4-octylphenyl)-5-(4-ethoxyphenyl)pyridin
2-(2-Fluor-4-octylphenyl)-5-(4-propoxyphenyl)pyridin
2-(2-Fluor-4-octylphenyl)-5-(4-butoxyphenyl)pyridin
2-(2-Fluor-4-octylphenyl)-5-(4-pentoxyphenyl)pyridin
2-(2-Fluor-4-octylphenyl)-5-(4-hexoxyphenyl)pyridin
2-(2-Fluor-4-octylphenyl)-5-(4-heptoxyphenyl)pyridin
2-(2-Fluor-4-octylphenyl)-5-(4-octoxyphenyl)pyridin
2-(2-Fluor-4-octylphenyl)-5-(4-nonoxyphenyl)pyridin

### Beispiel 3

Ein Gemisch aus 2-(3- Nitro-4-cyanophenyl)-5-heptylpyridin (herstellbar aus dem bekannten 2-(4-Bromphenyl)-5-heptylpyridin durch Nitrierung und anschließende Substitution von Br durch CN mittels CuCN) und Cäsiumfluorid in 1,3-Dimethyltetrahydro-2(1H)-pyrimidinon wird 4 Stunden auf 110 °C erhitzt.

Nach dem Abkühlen gießt man auf Eis/Wasser und extrahiert mit Dichlormethan. Die übliche Aufarbeitung durch Chromatographie und Kristallisation ergibt 2-(3-Fluor-4-cyanophenyl)-5-heptylpyridin.

Analog werden hergestellt:
2-(3-Fluor-4-cyanophenyl)-5-methylpyridin
2-(3-Fluor-4-cyanophenyl)-5-ethylpyridin
2-(3-Fluor-4-cyanophenyl)-5-propylpyridin
2-(3-Fluor-4-cyanophenyl)-5-butylpyridin
2-(3-Fluor-4-cyanophenyl)-5-pentylpyridin
2-(3-Fluor-4-cyanophenyl)-5-hexylpyridin
2-(3-Fluor-4-cyanophenyl)-5-octylpyridin
2-(3-Fluor-4-cyanophenyl)-5-nonylpyridin
2-(3-Fluor-4-cyanophenyl)-5-decylpyridin

## Patentansprüche (Patentansprüche für folgende(n) Vertragsstaat(en): CH, DE, GB, LI)

1. Heterocyclische Verbindungen der Formel I 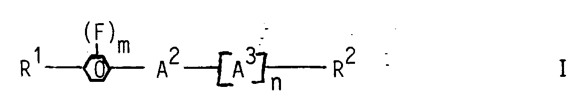 worin
R¹ und R² jeweils unabhängig voneinander eine Alkylgruppe mit 1-15 C-Atomen, worin auch eine oder zwei nicht benachbarte CH₂-Gruppen durch O-Atome und/oder -CO-Gruppen und/oder -O-CO-Gruppen und/oder -CO-O-Gruppen und/oder -O-COO-Gruppen und/oder -CHCN-und/oder -CH-Halogen-Gruppen ersetzt sein können, einer der Reste R¹ und R² auch F, Cl, Br oder CN,
m 1 oder mehr
A² eine Pyrazin-2,5-diylgruppe (Pa) oder eine Pyridin-2,5-diylgruppe (Py),
A³ eine 1,4-Cyclohexylengruppe oder eine 1,4-Phenylengruppe, die gegebenenfalls auch durch F substituiert sein kann,
n 0 oder 1
bedeutet, mit der Maßgabe, daß Verbindungen der Formel 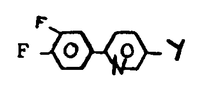 worin Y eine Alkylgruppe mit 1 bis 10 C-Atomen bedeutet, augeschlossen sind.

2. Verbindungen nach Anspruch 1, gekennzeichnet durch die Teilformeln I a and I c

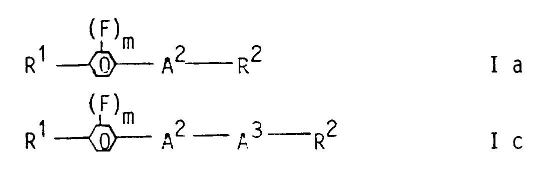

3. Verbindungen nach Anspruch 1 oder 2, dadurch gekennzeichnet, daß A² eine Pyridin-2,5-diylgruppe ist.

4. Verbindungen nach Anspruch 3, dadurch gekennzeichnet, daß n1 ist.

5. 2-(3-Fluor-4-cyanopheny1)-5-methylpyridin
2-(3-Fluor-4-cyanophenyl)-5-ethylpyridin
2-(3-Fluor-4-cyanophenyl)-5-propylpyridin
2-(3-Fluor-4-cyanophenyl)-5-butylpyridin
2-(3-Fluor-4-cyanophenyl)-5-pentylpyridin
2-(3-Fluor-4-cycanophenyl)-5-hexylpyridin
2-(3-Fluor-4-cyanophenyl)-5-heptylpyridin
2-(3-Fluor-4-cyanophenyl)-5-octylpyridin
2-(3-Fluor-4-cyanophenyl)-5-nonylpyridin
2-(3-Fluor-4-cyanophenyl)-5-decylpyridin

6. Verwendung der Verbindungen der Formel I nach Anspruch 1 als Komponenten flüssigkristalliner Phasen.

7. Flüssigkristalline Phase mit mindestens zwei flüssigkristallinen Komponenten, dadurch gekennzeichnet, daß mindestens eine Komponente eine Verbindung der Formel I ist.

8. Flüssigkristallanzeigeelement, dadurch gekennzeichnet, daß es eine Phase nach Anspruch 7 enthält.

9. Elektrooptisches Anzeigeelement nach Anspruch 8, dadurch gekennzeichnet, daß es als Dielektrikum eine Phase nach Anspruch 7 enthält.

## Patentansprüche (Patentansprüche für folgende(n) Vertragsstaat(en): FR, IT, NL)

1. Heterocyclische Verbindungen der Formel I 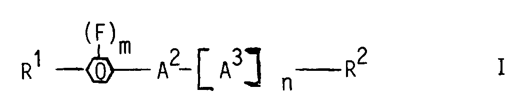 worin
R¹ und R² jeweils unabhängig voneinander eine Alkylgruppe mit 1-15 C-Atomen, worin auch eine oder zwei nicht benachbarte CH₂-Gruppen durch O-Atome und/oder -CO-Gruppen und/oder -O-CO-Gruppen und/oder -CO-O-Gruppen und/oder -O-COO-Gruppen und/oder -CHCN-und/oder -CH-Halogen-Gruppen ersetzt sein können, einer der Reste R¹ und R² auch F, Cl, Br oder CN,
m 1 oder mehr,
A² eine Pyrazin-2,5-diylgruppe (Pa) oder eine Pyridin-2,5-diylgruppe (Py),
A³ eine 1,4-Cyclohexylengruppe oder eine 1,4-Phenylengruppe, die gegebenenfalls auch durch F substituiert sein kann,
n 0 oder 1
bedeutet.

2. Verbindungen nach Anspruch 1, gekennzeichnet durch die Teilformeln I a and I c

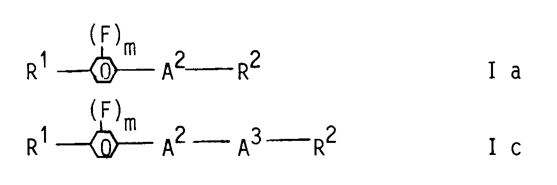

3. Verbindungen nach Anspruch 1 oder 2, dadurch gekennzeichnet, daß A² eine Pyridin-2,5-diylgruppe ist.

4. Verbindungen nach Anspruch 3, dadurch gekennzeichnet, daß n1 ist.

5. 2-(3-Fluor-4-cyanophenyl)-5-methylpyridin
2-(3-Fluor-4-cyanophenyl)-5-ethylpyridin
2-(3-Fluor-4-cyanophenyl)-5-propylpyridin
2-(3-Fluor-4-cyanophenyl)-5-butylpyridin
2-(3-Fluor-4-cyanophenyl)-5-pentylpyridin
2-(3-Fluor-4-cyanophenyl)-5-hexylpyridin
2-(3-Fluor-4-cyanophenyl)-5-heptylpyridin
2-(3-Fluor-4-cyanophenyl)-5-octylpyridin
2-(3-Fluor-4-cyanophenyl)-5-nonylpyridin
2-(3-Fluor-4-cyanophenyl)-5-decylpyridin

6. Verwendung der Verbindungen der Formel I nach Anspruch 1 als Komponenten flüssigkristalliner Phasen.

7. Flüssigkristalline Phase mit mindestens zwei flüssigkristallinen Komponeten, dadurch gekennzeichnet, daß mindestens eine Komponente eine Verbindung der Formel I ist.

8. Flüssigkristallanzeigeelement, dadurch gekennzeichnet, daß es eine Phase nach Anspruch 7 enthält.

9. Elektrooptisches Anzeigeelement nach Anspruch 8, dadurch gekennzeichnet, daß es als Dielektrikum eine Phase nach Anspruch 7 enthält.

## Claims (Claims for the following Contracting State(s): CH, DE, GB, LI)

1. Heterocyclic compounds of the formula I 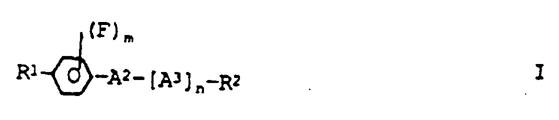 wherein
R¹ and R² are each independently of the other an alkyl group of 1-15 C atoms, wherein one or two non-adjacent CH₂ groups can also be replaced by 0 atoms and/or -CO- groups and/or -O-CO- groups and/or -CO-O- groups and/or -O-COO- groups and/or -CHCN- and/or -CH-halogen groups, and one of the radicals R¹ and R² is also F, Cl, Br or CN,
m is 1 or more,
A² is a pyrazine-2,5-diyl group (Pa) or a pyridine-2,5-diyl group (Py),
A³ is a 1,4-cyclohexylene group or a 1,4-phenylene group which, if appropriate, may also be substituted by F, and
n is 0 or 1,
with the proviso that compounds of the formula 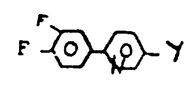 wherein Y is an alkyl group of 1 to 10 C atoms are excluded.

2. Compounds according to Claim 1, characterized by the partial formulae I a and I c

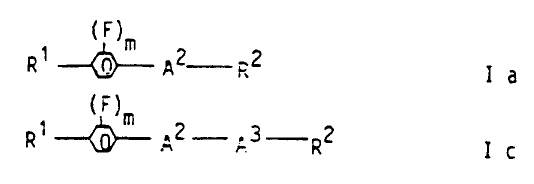

3. Compounds according to Claim 1 or 2, characterized in that A² is a pyridine-2,5-diyl group.

4. Compounds according to Claim 3, characterized in that n is 1.

5. 2-(3-Fluoro-4-cyanophenyl)-5-methylpyridine
2-(3-Fluoro-4-cyanophenyl)-5-ethylpyridine
2-(3-Fluoro-4-cyanophenyl)-5-propylpyridine
2-(3-Fluoro-4-cyanophenyl)-5-butylpyridine
2-(3-Fluoro-4-cyanophenyl)-5-pentylpyridine
2-(3-Fluoro-4-cyanophenyl)-5-hexylpyridine
2-(3-Fluoro-4-cyanophenyl)-5-heptylpyridine
2-(3-Fluoro-4-cyanophenyl)-5-octylpyridine
2-(3-Fluoro-4-cyanophenyl)-5-nonylpyridine
2-(3-Fluoro-4-cyanophenyl)-5-decylpyridine

6. Use of the compounds of the formula I according to Claim 1 as components of liquid-crystalline phases.

7. Liquid-crystalline phase containing at least two liquid-crystalline components, characterized in that at least one component is a compound of the formula I.

8. Liquid crystal display element, characterized in that it contains a phase according to Claim 7.

9. Electro-optical display element according to Claim 8, characterized in that it contains as dielectric a phase according to Claim 7.

## Claims (Claims for the following Contracting State(s): FR, IT, NL)

1. Heterocyclic compounds of the formula I 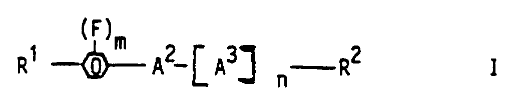 wherein
R¹ and R² are each independently of the other an alkyl group of 1-15 C atoms, wherein one or two non-adjacent CH₂ groups can also be replaced by 0 atoms and/or -CO- groups and/or -O-CO- groups and/or -CO-O- groups and/or -O-COO- groups and/or -CHCN- and/or -CH-halogen groups, and one of the radicals R¹ and R² is also F, Cl, Br or CN,
m is 1 or more,
A² is a pyrazine-2,5-diyl group (Pa) or a pyridine-2,5-diyl group (Py),
A³ is a 1,4-cyclohexylene group or a 1,4-phenylene group which, if appropriate, may also be substituted by F, and
n is 0 or 1.

2. Compounds according to Claim 1, characterized by the partial formulae I a and I c

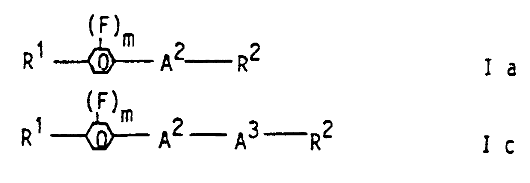

3. Compounds according to Claim 1 or 2, characterized in that A² is a pyridine-2,6-diyl group.

4. Compounds according to Claim 3, characterized in that n is 1.

5. 2-(3-Fluoro-4-cyanophenyl)-5-methylpyridine
2-(3-Fluoro-4-cyanophenyl)-5-ethylpyridine
2-(3-Fluoro-4-cyanophenyl)-5-propylpyridine
2-(3-Fluoro-4-cyanophenyl)-5-butylpyridine
2-(3-Fluoro-4-cyanophenyl)-5-pentylpyridine
2-(3-Fluoro-4-cyanophenyl)-5-hexylpyridine
2-(3-Fluoro-4-cyanophenyl)-5-heptylpyridine
2-(3-Fluoro-4-cyanophenyl)-5-octylpyridine
2-(3-Fluoro-4-cyanophenyl)-5-nonylpyridine
2-(3-Fluoro-4-cyanophenyl)-5-decylpyridine

6. Use of the compounds of the formula I according to Claim 1 as components of liquid-crystalline phases.

7. Liquid-crystalline phase containing at least two liquid-crystalline components, characterized in that at least one component is a compound of the formula I.

8. Liquid crystal display element, characterized in that it contains a phase according to Claim 7.

9. Electro-optical display element according to Claim 8, characterized in that it contains as dielectric a phase according to Claim 7.

## Revendications (Revendications pour l'(les) Etat(s) contractant(s) suivant(s): CH, DE, GB, LI)

1. Composés hétérocycliques de formule I 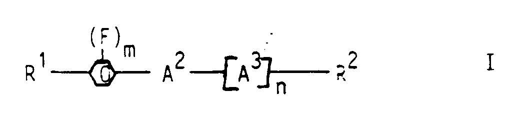 dans laquelle
R¹ et R² représentent chaque fois indépendamment l'un de l'autre un groupe alkyle de 1-15 atomes de C, dans lequel aussi un ou deux groupes CH₂ non contigüs peuvent être remplacés par des atomes de 0 et/ou des groupes -CO- et/ou des groupes -O-CO- et/ou des groupes -CO-O- et/ou des groupes -O-COO- et/ou des groupes -CHCN- et/ou des groupes -CH-halogène, l'un des restes R¹ et R² peut être aussi F, Cl, Br ou CN,
m représente 1 ou plus,
A² représente un groupe pyrazine-2,5-diyle (Pa) ou un groupe pyridine-2,5-diyle (Py),
A³ représente un groupe 1,4-cyclohexylène ou un groupe 1,4-phénylène, qui peut être aussi substitué par F le cas échéant,
n représente 0 ou 1
à la condition qu'on exclue les composés de formule 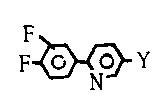 dans laquelle Y représente un groupe alkyle de 1 à 10 atomes de C.

2. Composés selon la revendication 1, caractérisés par les formules partielles Ia et Ic

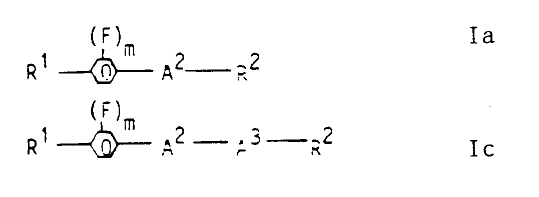

3. Composés selon la revendication 1 ou 2, caractérisés en ce que A² est un groupe pyridine-2,5-diyle.

4. Composés selon la revendication 3, caractérisés en ce que n est 1.

5. 2-(3-fluoro-4-cyanophényl)-5-méthylpyridine
2-(3-fluoro-4-cyanophényl)-5-éthylpyridine
2-(3-fluoro-4-cyanophényl)-5-propylpyridine
2-(3-fluoro-4-cyanophényl)-5-butylpyridine
2-(3-fluoro-4-cyanophényl)-5-pentylpyridine
2-(3-fluoro-4-cyanophényl)-5-hexylpyridine
2-(3-fluoro-4-cyanophényl)-5-heptylpyridine
2-(3-fluoro-4-cyanophényl)-5-octylpyridine
2-(3-fluoro-4-cyanophényl)-5-nonylpyridine
2-(3-fluoro-4-cyanophényl)-5-décylpyridine

6. Utilisation des composés de formule I selon la revendication 1 comme composants de phases de cristaux liquides.

7. Phase de cristaux liquides avec au moins deux composants de cristaux liquides, caractérisée en ce qu'au moins un composant est un composé de formule I.

8. Elément d'affichage à cristaux liquides, caractérisé en ce qu'il contient une phase selon la revendication 7.

9. Elément d'affichage électrooptique selon la revendication 8, caractérisé en ce qu'il contient comme diélectrique une phase selon la revendication 7.

## Revendications (Revendications pour l'(les) Etat(s) contractant(s) suivant(s): FR, IT, NL)

1. Composés hétérocycliques de formule I 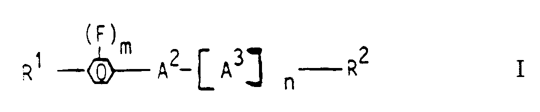 dans laquelle
R¹ et R² représentent chaque fois indépendamment l'un de l'autre un groupe alkyle de 1-15 atomes de C, dans lequel aussi un ou deux groupes CH₂ non contigüs peuvent être remplacés par des atomes de O et/ou des groupes -CO- et/ou des groupes -O-CO- et/ou des groupes -CO-O- et/ou des groupes -O-COO- et/ou des groupes -CHCN- et/ou des groupes -CH-halogène, l'un des restes R¹ et R² peut ête aussi F, Cl, Br ou CN,
m représente 1 ou plus
A² représente un groupe pyrazine-2,5-diyle (Pa) ou un groupe pyridine-2,5-diyle (Py),
A³ représente un groupe 1,4-cyclohexylène ou un groupe 1,4-phénylène, qui peut être aussi substitué par F le cas échéant,
n représente 0 ou 1.

2. Composés selon la revendication 1, caractérisés par les formules partielles Ia et Ic

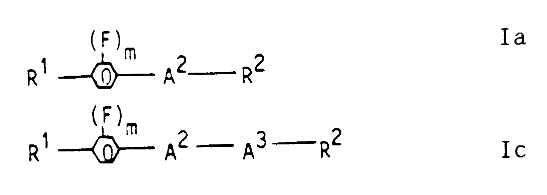

3. Composés selon la revendication 1 ou 2, caractérisés en ce que A₂ est un groupe pyridine-2,5-diyle.

4. Composés selon la revendication 3, caractérisés en ce que n est 1.

5. 2-(3-fluoro-4-cyanophényl)-5-méthylpyridine
2-(3-fluoro-4-cyanophényl)-5-éthylpyridine
2-(3-fluoro-4-cyanophényl)-5-propylpyridine
2-(3-fluoro-4-cyanophényl)-5-butylpyridine
2-(3-fluoro-4-cyanophényl)-5-pentylpyridine
2-(3-fluoro-4-cyanophényl)-5-hexylpyridine
2-(3-fluoro-4-cyanophényl)-5-heptylpyridine
2-(3-fluoro-4-cyanophényl)-5-octylpyridine
2-(3-fluoro-4-cyanophényl)-5-nonylpyridine
2-(3-fluoro-4-cyanophényl)-5-décylpyridine.

6. Utilisation des composés de formule I selon la revendication 1 comme composants de phases de cristaux liquides.

7. Phase de cristaux liquides avec au moins deux composants de cristaux liquides, caractérisée en ce qu'au moins un composant est un composé de formule I.

8. Elément d'affichage à cristaux liquides, caractérisé en ce qu'il contient une phase selon la revendication 7.

9. Elément d'affichage électrooptique selon la revendication 8, caractérisé en ce qu'il contient comme diélectrique une phase selon la revendication 7.
